# EUROPÄISCHE PATENTSCHRIFT

(11) **EP 0 868 188 B1**
(45) Veröffentlichungstag und Bekanntmachung des Hinweises auf die Patenterteilung: **09.07.2003**
(21) Anmeldenummer: 96946221.7
(22) Anmeldetag: 20.12.1996
(51) Int. Cl.: A61K 31/57, A61K 31/565, A61P 15/18

(54) **KIT ZUR KONTRAZEPTION BEI WEIBLICHEN SÄUGERN, BESTEHEND AUS EINER KOMBINATION VON GESTAGEN UND ESTROGEN**
KIT FOR FEMALE MAMMALS, COMPRISING A COMBINATION OF GESTAGEN AND OESTROGEN
KIT A USAGE CONTRACEPTIF POUR MAMMIFERES FEMELLES, CONSTITUE D'UNE COMBINAISON DE GESTAGENE ET D'OESTROGENE

(30) Priorität: 23.12.1995 DE 19549264
(43) Veröffentlichungstag der Anmeldung: 07.10.1998
(73) Patentinhaber: SCHERING AKTIENGESELLSCHAFT, 13353 Berlin (DE)
(72) Erfinder: ENDRIKAT, Jan, D-12207 Berlin (DE); DÜSTERBERG, Bernd, D-12307 Berlin (DE); REILHAC, Pia, F-44000 Nantes (FR)
(86) Internationale Anmeldenummer: DE9602486
(87) Internationale Veröffentlichungsnummer: WO97023228

(56) Entgegenhaltungen:
- EP-A- 0 368 373

## Beschreibung

Die vorliegende Erfindung betrifft ein Kit zur Kontrazeption bei weiblichen Säugern.

Seit den 60iger Jahren sind hormonale Kontrazeptiva einerseits als sogenannte Kombinationspräparate und Stufenpräparate und andererseits als Sequenzpräparate bekannt. Alle diese Präparate hemmen die Ovulation und bewirken regelmäßige menstruelle Blutungen (Abbruchblutungen).
Die meisten hormonalen Kontrazeptiva enthalten ein Estrogen und ein Gestagen (Tabelle 1).

Die verschiedenen Typen hormonaler Kontrazeptiva.

| Bezeichnung | Zusammensetzung |
|---|---|
| Kombinationspräparat (Einphasenpräparat) abgestuftes Kombinationspräparat | Estrogen und Gestagen |
| Sequenzpräparat | Estrogen (1. Phase) und Estrogen/Gestagen (2. Phase) |
| Minipille | Gestagen |
| Postkoitalpille | Gestagen |

Kombinationspräparate sind gekennzeichnet durch gleichbleibende Dosierung der beiden hormonellen Komponenten (Estrogen/Gestagen). Durch die gleichzeitig Gabe der Gestagen- und der Estrogenkomponente vom ersten Applikationstag an, weisen Kombinationspräparate eine hohe kontrazeptive Zuverlässigkeit auf. Bei allen Formen der Kombinationspräparate wird der ovulatorische LH-Gipfel zuverlässig supprimiert, so dass sowohl die Ovulation als auch die Corpus-luteum-Bildung unterdrückt werden [Elstein, M. et a.: Studies on low dose oral contraceptives: cervicalmucus and plasma hormone changes in relation to circulating d-norgestrel and 17-ethinyl estradiol concentrations. Fertil.Sterl 27:892 (1976)]. Die frühe sekretorische Transformation des wenig entwickelten Endomeriums kann insbesondere während der ersten Einnahmezyklen das Auftreten von Spottings (Zwischenblutungen) zur Folge haben.

Um die Gestagendosis gering zu halten wurden sogenannte abgestufte Kombinationspräparate entwickelt. Hierbei unterscheidet man zwischen den Zwei- und Dreistufenpräparaten. Die Zweistufenpräparate zeichnen sich dadurch aus, daß die Gestagengabe in zwei Phasen untergliedert wird. In der ersten Phase (11 Tage) wird eine geringere Gestagendosis als in der zweiten Phase, bei gleichbleibender Estrogendosis, verabreicht. Bei den Dreistufenpräparaten wurde das Prinzip der abgestuften Kombinationspräparate weiter verfeinert, es handelt sich um eine Modifikation des Zweistufenpräparates. Hier wird die Gestagendosis in drei Phasen unterteilt: die erste Phase enthält eine geringe Gestagendosis, die während der folgenden zwei Phasen erhöht wird, während die Estrogendosis entweder konstant über alle drei Phasen ist oder während der zweiten Phase erhöht wird.
Sequenzpräparate zeichnen sich dadurch aus, daß sie in den ersten 7 bis maximal 11 Tagen der Anwendung eine reine Estrogenkomponente und erst in den darauf folgenden 10 bis maximal 14 Tagen ein Gestagenkomponente enthalten. Der Einfluß dieser Präparate auf das Endometrium kommt den pysiologischen zyklusabhängigen Hormonaleneinfluß sehr nahe.
Die kontrazeptive Zuverlässigkeit der typischen Sequenzpräparate beruht in der ersten Phase nur auf der gonadotropinhemmenden Wirkung des Estrogens, während das in der zweiten Phase zusätzlich eingenommene Gestagen vorwiegend zur sekretorischen Umwandlung des Endometriums und zur regelmäßigen Auslösung einer Entzugsblutung dient.
Die meisten oralen Kontrazeptiva werden über einen Zeitraum von 21 Tagen, gefolgt von 7 Tagen Placebos oder Pillen-freien Tagen, verabreicht, so daß ein normaler Zyklus immitiert wird.

Des weiteren sind reine Gestagenpräparate bekannt.

In frühen Untersuchungen konnte gezeigt werden, daß schon sehr geringe Dosen des Gestagens Chlormadinonacetat einen Kontrazeptionsschutz gewährten, obwohl es durch die geringe Gestagendosis nicht immer zu einer Ovulationshemmung kommt (Martinez-Manautou, J., J. Giner-Velasquez, V. Gallegos-Cortès, J. Casasola, R. Aznar, H. Rudel: Fertility control with microdose of progestogen. In C. Gual: Proc. VIth Pan-Amer. Conf. Endocr. Mexico City 1965. Exerpta med. (amst.) Int. Congr. Ser. No.112, p 157-165; Rudel, H. W., J. Martinez-Manautou, M Maqueo-Topete: The role of progestetogens in the hormonal control of fertility. Fert. and Sterl. 16 (1965) 158-169].

Die Verwendung reiner Gestagenpräparate zur Kontrazeption wurde wieder interessant, nachdem sich heraustellte, daß die estrogene Komponente: für einige unerwünschte Begleiterscheinungen (Kopfschmerzen; Übelkeit, Gewichtszunahme etc.) und vor allem für gefährliche Komplikationen wie thromboembolische Erkrankungen verantwortlich gemacht werden könnte [Daniel, D. G., Campell, A. C. Turnbull:
Perperalthromboembolism and suppression of lactation. Lancet 1967/II, 287-289J.

Aufgrund der geringen Dosierung erhielten die reinen. Gestagenpräparate die Bezeichnung Minipille. Bei den bisher eingeführten Minipillen handelt es sich ausnahmslos um Abkömmlinge des 19-Nortestosterons: Norethisteron, Lynestrenol, Levonorgestrel.
Die Minipillen werden im Gegensatz zu den Estrogen-/Gestagenpräparaten durchgängig, ohne Rücksicht auf den Zeitpunkt der Blutung verabreicht, weil man vermutete, daß die Unzuverläßigkeit der bisher bekannten reinen Gestagenpräparate zu verhindern sei, wenn die Applikationsdauer verlängert würde.
Die bisher beschriebenen reinen Gestagenpräparate weisen eine nicht sehr hohe kontrazeptive Zuverlässigkeit auf, was darauf zuruckzuführen ist, daß die Ovulation nicht immer regelmaßig inhibiert wird [Vessey et al.: Progestogen-only oral contrception. Findings in large prospective study with special reference to effectivness, Brit.J. Family Planning, 292: 526-30 (1986)J. Im allgemeinen kann man damit rechnen, daß der Anteil der anovulatorischen Zyklen unter dem Einfluß dieser niedrig dosierten Gestagene nur zwischen 15% und 40% liegt [Chi, I.: The safety and efficacy of progestin-only oral. contraceptives. An epidemiologic perspective. Contraception 47 (1993) 1-21).
Die Patenanmeldung EP A 0 491 443 offenbart ein reines Gestagenpräparat, bei welchem die Gestagene Desogestrel und 3-Ketodesogestrel in einer Tagesdosis von 70 bis 80 µg verabreicht werden. Diese Dosierungen führen bei Fast allen Frauen bereits zur Hemmung der Ovulation.

Werden Gestagene allein in ovulationshemmenden Dosis verabreicht besteht jedoch die Gefahr einer Amenorrhö und bei längerer Verabreichung können weitere Symptome einer Hypoestrogenität auftreten.

Es besteht daher das Bedürfnis die Vorteile eines reinen Gestagenpräparates kombiniert mit einer sicheren Zykluskontrolle und regelmäßigen menstruationsähnlichen Blutungen bereitzustellen.

Es wurde nun gefunden, dass überraschenderweise ein kontrazeptionelles Kit, enthaltend mindestens 28 tägliche Dosierungseinheiten mit einer ersten Phase, bestehend aus mindestens 18 bis 23 ersten täglichen Dosierungseinheiten eines Gestagens in einer ovulationshemmenden Dosis und einer zweiten Phase, bestehend aus mindestens 5 bis 10 täglichen Dosierungseinheiten eines Gestagens in einer ovulationshemmenden Dosis, in Kombination mit einem natürlichen Estrogen, zu einer optimalen Zykluskontrolle und regelmäßigen menstruationsähntichen Blutungen führt.

In einer weiteren Ausführungsform betrifft die Erfindung ein kontrazeptives Kit, enthaltend 28 tägliche Dosierungseinheiten mit einer ersten Phase, bestehend aus 18 bis 23 ersten täglichen Dosierungseinheiten eines Gestagens in einer ovulationshemmenden Dosis und einer zweiten Phase, bestehend aus 5 bis 10 zweiten täglichen Dosierungseinheiten eines Gestagens in einer ovutationshemmenden Dosis, in Kombination mit einem natürlichen Estrogen.

In einer weiteren Ausführungsform betrifft die Erfindung die bereits beschriebenen Kits, wobei das natürliche Estrogen über 10 Tage des letzten Drittels der sequentiellen Gabe verabreicht wird.

In einer bevorzugten Ausführungsform des erfinderischen kontrazeptionellen Kits ist dieses für die Frau vorgesehen.

Die Applikation des Gestagens oder des natürlichen Estrogens gemäß vorliegender Erfindung kann so erfolgen, dass beide Komponenten transdermal appliziert werden oder aber auch, dass beispielsweise das Gestagen transdermal appliziert wird und die Applikation des natürlichen Estrogens oral erfolgt oder umgekehrt das natürliche Estrogen transdermal appliziert wird und das Gestagen oral.

Vorzugsweise wird in allen Ausführungsformen der Erfindung das Gestagen aus der Gruppe der Verbindungen:
Gestoden,
Progesteron,
Levonorgestrel,
Cyproteronacetat,
Chlormadinonacetat,
Drospirenon (Dihydrospirorenon),
Norethisteron,
Norethisteronacetat,
Norgestimat,
Desogestrel,
3-Ketodesogestrel,
Dienogest
oder einem Gemisch hieraus ausgewählt.

In einer besonderen Ausführungsform ist das Gestagen in einer täglichen Dosierung von:
0,05-0,2 mg Levonorgestrel,
0,05-0,15 mg Gestoden
oder einer bioequivalenten Dosierung eines anderen Gestagens enthalten.

In einer besonderen Ausführungsform ist das Gestagen Levonorgestrel in einer täglichen Dosierung von 0,1 mg oder Gestoden in einer täglichen Dosierung von 0,075 mg enthalten.

Das erfinderische Kit kombiniert die Vorteile einer reinen Gestagengabe miteiner sicheren Zykluskontrolle und regelmäßigen menstruationsähnlichen Blutungen. Das Gestagen gewährleistet die kontrazeptionelle Wirkung, während durch das natürliche Estrogen das Endometrium aufgebaut wird und es am Ende der Kombinationsphase jeweils zu menstruationsähnlichen Blutungen kommt.

Dieses Regime weist gegenüber den bisher bekannten Kits zur oralen Kontrazeption folgende Vorteile auf:
- Die Ovulation wird durch eine niedrige aber ausreichend hohe tägliche Gestagendosis effektiv gehemmt.
- Durch die sequentielle Gabe des natürlichen Estrogens wird eine gute Zykluskontrolle gewährleistet.
- Das vorliegende erfinderische Kontrazeptivum ist durch die Verwendung eines natürlichen Estrogens auch für Frauen in der Prämenopause gut verträglich und führt, insbesondere am Knochen, zu positiven Effekten.
- Durch da Verwenden eines natürlichen Estrogens ist eine gute allgemeine und insbesondere Leber-Verträglichkeit gewährleistet.
- Es kommt zu deutlich weniger Ethinylestradiol bedingten Nebenwirkungen

### Beispiele:

### Beispiele für die Ausgestaltung des erfinderischen kontrazeptionellen Kits

### Beispiel 1.

| | |
|---|---|
| | 10tägige Gabe von 2,5 mg Estradiol pro Tag |
| 28tägige Gabe von 0,1 mg Levonorgestrel pro Tag | |

### Beispiel 2.

| | |
|---|---|
| | 8 tägige Gabe von 2,5 mg Estradiol pro Tag |
| 28tägige Gabe von 0,1 mg Levonorgestrel pro Tag | |

### Beispiel 3.

| | |
|---|---|
| | 10tägige Gabe von 2,5 mg Estradiol pro Tag |
| 56tägige Gabe von 0, mg Levonorgestrel pro Tag | |

### Beispiel 4.

| | |
|---|---|
| | 10tägige Gabe von 2,5 mg Estradiol pro Tag |
| 84tägige Gabe von 0,1 mg Levonorgestrel pro Tag | |

### Beispiel 5.

| | |
|---|---|
| | 10tägige Gabe von 2,5 mg Estradiol pro Tag |
| 28tägige Gabe von 0,075 mg Gestoden pro Tag | |

### Beispiel 6.

| | |
|---|---|
| | 8tägige Gabe von 2,5 mg Estradiol pro Tag |
| 28tägige Gabe von 0,075 mg Gestoden pro Tag | |

### Beispiel 7.

| | |
|---|---|
| | 10tägige Gabe von 2,5 mg Estradiol pro Tag |
| 56tägige Gabe von 0,075 mg Gestoden pro Tag | |

### Beispiel 8.

| | |
|---|---|
| | 10tägige Gabe von 2,5 mg Estradiol pro Tag |
| 84tägige Gabe von 0,075 mg Gestoden pro Tag | |

### Beispiele für die Ausgestaltung des kontrazeptionellen Kit

### Beispiel 1

| MO | DI | MI | DO | FR | SA | SO |
|---|---|---|---|---|---|---|
| | | | | | | |
| • | • | • | • | • | • | • |
| • | • | • | • | • | • | • |
| • | • | • | • | • | • | • |
| ○ | ○ | ○ | ○ | ○ | ○ | ○ |
| • = Gestagen-Dosierungseinheit (z.B. Levonorgestrel 0.1 mg oder Gestoden 0,075 mg) o = Gestagen- und Estrogen-Dosierungseinheit (z.B.Levonorgestrel 0.1 mg/Estradiol 2,5 mg oder Gestoden 0,075 mg/Estradiol 2,5 mg)) | | | | | | |

### Beipiel 2.

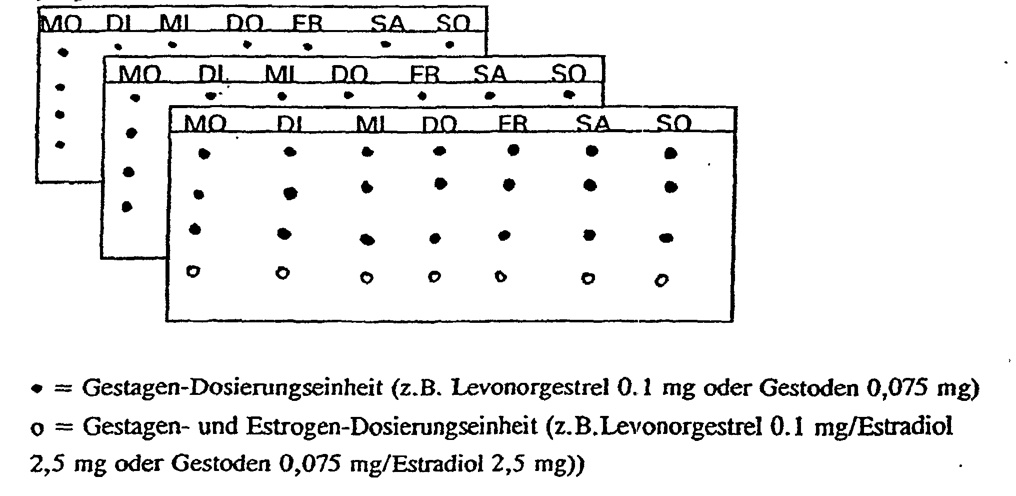

Weitere Ausführungsformen des erfinderischen Kits sind der Beschreibung zu entnehmen.

Das Gestagen und das Estrogen kann im erfindungsgemäßen Kit so vorliegen, dass deren Applikation lokal, topisch, enteral, transdermal oder parenteral erfolgen kann.

Für die bevorzugte orale Applikation kommen insbesondere Tabletten, Dragees, Kapseln, Pillen, Suspensionen oder Lösungen in Frage, die in der üblichen Weise mit den in der Galenik gebräuchlichen Zusätzen und Trägersubstanzen hergestellt werden können.

Für die lokale oder topische Anwendung kommen beispielsweise Vaginalzäpfchen, Vaginalgels, Implantate, Vaginalringe oder transdermale Systeme wie Hautpflaster in Frage.

Erfolgt die Applikation durch ein Implantat, einen Vaginalring oder ein transdermales System, so müssen diese Applikationssysteme derart beschaffen sein, dass durch sie eine der täglichen oralen Dosis wirkequivalente Dosis für die jeweilige Applikationsform täglich freigesetzt wird.

Für eine transdermale Applikation durch ein Hauptpflaster eignen sich insbesondere die folgenden Gestagen: Gestoden, Levonorgestrel, 3-Ketodesogestrel oder ein Gemisch hieraus und als natürliches Estrogen: Estradiol in einer Konzentration von 0,025-0,25 mg Freisetzungsrate pro Tag. Die Freisetzungsrate pro Tag für die transdermal durch ein Hautpflaster applizierten Gestagene entspricht den angegebenen Tagesdosiskonzentrationen.

Die Bestimmung wirkequivalenter Dosismengen verschiedener Gestagene und natürlicher Estrogene wird nach bekannten Methoden vorgenommen; nähere Einzelheiten finden sich beispielsweise in den beiden Artikeln »Probleme der Dosisfindung: Sexualhormone"; F. Neumann et al. In "Arzneimittelforschung" (Drug Research) 27, 2a, 296 - 318 (1977) sowie "Aktuelle Entwicklungen in der hormonalen Kontrazeption"; H. Kuhl in "Gynäkologe" 25: 231 - 240 (1992).

## Patentansprüche

1. Kontrazeptionelles Kit, enthaltend mindestens 28 tägliche Dosierungseinheiten mit
(a) einer ersten Phase, bestehend aus mindestens 18 bis 23 ersten täglichen Dosierungseinheiten eines Gestagens in einer ovulationshemmenden Dosis und
(b) einer zweiten Phase, bestehend aus mindestens 5 bis 10 zweiten täglichen Dosierungseinheiten eines Gestagens in einer ovulationshemmenden Dosis, in Kombination mit einem natürlichen Estrogen.

2. Kontrazeptionelles Kit, enthaltend 28 tägliche Dosierungseinheiten mit
(a) einer ersten Phase, bestehend aus 18 bis 23 ersten täglichen Dosierungseinheiten eines Gestagens in einer ovulationshemmenden Dosis
und
(b) einer zweiten Phase, bestehend aus 5 bis 10 zweiten täglichen Dosierungseinheiten eines Gestagens in einer ovulationshemmenden Dosis, in Kombination mit einem natürlichen Estrogen.

3. Kontrazeptionelles Kit nach Anspruch 1 oder 2, mit dem das natürliche Estrogen über 10 Tage des letzten Drittels der sequentiellen Gabe verabreicht wird.

4. Kontrazeptionelles Kit nach einem der Ansprüche 1 bis 3, in dem das Gestagen aus der Gruppe der Verbindungen:
Gestoden,
Progesteron,
Levonorgestrel,
Cyproteronacetat,
Chlormadinonacetat,
Drospirenon (Dihydrospirorenon),
Norethisteron,
Norethisteronacetat,
Norgestimat,
Desogestrel,
3-Ketodesogestrel,
Dienogest
oder einem Gemisch hieraus ausgewählt ist.

5. Kontrazeptionelles Kit nach Anspruche 4, worin das Gestagen in einer täglichen Dosierung
0,05-0,2 mg Levonorgestrel.
0,05-0,15 Gestogen
oder einer bioequivalenten Dosierung eines anderen Gestagens enthalten ist.

6. Kontrazeptionelles Kit nach einem der vorgehenden Ansprüche, bei dem die Applikation des Gestagens oral und die Applikation des natürlichen Estrogens transdermal erfolgt.

7. Kontrazeptionelles Kit nach einem der vorhergehenden Ansprüche, bei dem die Applikation des Gestagens transdermal und die Applikation des natürlichen Estrogens oral erfolgt.

## Claims

1. Contraceptive kit, comprising at least 28 daily dosage units having
(a) a first phase, consisting of at least 18 to 23 first daily dosage units of a progestogen in an ovulation-inhibiting dose
and
(b) a second phase, consisting of at least 5 to 10 second daily dosage units of a progestogen in an ovulation-inhibiting dose, in combination with a natural oestrogen.

2. Contraceptive kit, comprising 28 daily dosage units having
(a) a first phase, consisting of 18 to 23 first daily dosage units of a progestogen in an ovulation-inhibiting dose
and
(b) a second phase, consisting of 5 to 10 second daily dosage units of a progestogen in an ovulation-inhibiting dose, in combination with a natural oestrogen.

3. Contraceptive kit according to Claim 1 or 2, using which the natural oestrogen is administered over 10 days of the last third of the sequential administration.

4. Contraceptive kit according to one of Claims 1 to 3, in which the progestogen is selected from the group of compounds:
gestodene,
progesterone,
levonorgestrel,
cyproterone acetate,
chlormadinone acetate,
drospirenone (dihydrospirorenone),
norethisterone,
norethisterone acetate,
norgestimate,
desogestrel,
3-ketodesogestrel
dienogest
or a mixture thereof.

5. Contraceptive kit according to Claim 4, in which the progestogen is present in a daily dose of
0.05-0.2 mg of levonorgestrel,
0.05-0.15 mg of gestodene
or a bioequivalent dose of another progestogen.

6. Contraceptive kit according to one of the preceding Claims, in which the administration of the progestogen takes place orally and the administration of the natural oestrogen takes place transdermally.

7. Contraceptive kit according to one of the preceding Claims, in which the administration of the progestogen takes place transdermally and the administration of the natural oestrogen takes place orally.

## Revendications

1. Kit à usage contraceptif, contenant au minimum 28 unités de dosage journalières comprenant
(a) une première phase, constituée d'au moins 18 à 23 premières unités de dosage journalières d'un gestagène en une dose inhibant l'ovulation, et
(b) une deuxième phase, constituée d'au moins 5 à 10 deuxièmes unités de dosage journalières d'un gestagène en une dose inhibant l'ovulation, en combinaison avec un oestrogène naturel.

2. Kit à usage contraceptif, contenant 28 unités de dosage journalières comprenant
(a) une première phase, constituée de 18 à 23 premières unités de dosage journalières d'un gestagène en une dose inhibant l'ovulation, et
(b) une deuxième phase, constituée de 5 à 10 deuxièmes unités de dosage journalières d'un gestagène en une dose inhibant l'ovulation, en combinaison avec un oestrogène naturel.

3. Kit à usage contraceptif selon la revendication 1 ou 2, avec lequel l'oestrogène naturel est administré sur 10 jours du dernier tiers de l'administration séquentielle.

4. Kit à usage contraceptif selon l'une des revendications 1 à 3, dans lequel le gestagène est choisi dans le groupe des composés suivants:
Gestodène,
Progestérone,
Lévonorgestrel,
Acétate de cyprotérone,
Acétate de chlormadinone,
Drospirénone (dihydrospirorénone),
Noréthistérone,
Acétate de noréthistérone,
Norgestimat,
Désogestrel,
3-Cétodésogestrel,
Diénogest
ou d'un mélange de ceux-ci.

5. Kit à usage contraceptif selon la revendication 4, où le gestagène est contenu en une dose journalière de
0,05 à 0,2 mg de lévonorgestrel,
0,05 à 0,15 de gestogène
ou d'une dose bioéquivalente d'un autre gestagène.

6. Kit à usage contraceptif selon l'une des revendications qui précèdent, dans lequel l'application du gestagène est opérée par voie orale et l'application de l'oestrogène naturel par voie transdermique.

7. Kit à usage contraceptif selon l'une des revendications qui précèdent, dans lequel l'application du gestagène est opérée par voie transdermique et l'application de l'oestrogène naturel par voie orale.
